# DEMANDE DE BREVET EUROPEEN

(11) **EP 1 815 884 A2**
(43) Date de publication de la demande: **08.08.2007**
(21) Numéro de dépôt: 07100581.3
(22) Date de dépôt: 16.01.2007
(51) Int. Cl.: A61Q 17/04, A61K 8/49, A61K 8/37

(54) **Compositions contenant un filtre uv-b du type ester de l'acide cinnamique, un filtre uv-a du type dibenzoylmethane et un derive de s-triazine ; procede de photostabilisation**

(30) Priorité: 03.02.2006 FR 0650390
(71) Demandeur: L'Oréal, 75008 Paris (FR)
(72) Inventeur: Richard, Hervé, 93320, Les Pavillons sous Bois (FR); Candau, Didier, 91570, Bievres (FR)
(74) Mandataire: Miszputen, Laurent

(57) **Abrégé**

La présente invention est relative à une composition en particulier une composition cosmétique à usage topique contenant l'association d'un filtre UV-B du type ester de l'acide cinnamique, d'un filtre UV-A du type dérivé de dibenzoylméthane et d'une s-triazine possédant deux groupes para-aminobenzalmalonate encombrés et un groupe para-aminobenzoate de formule (I) particulière.

Elle concerne également un procédé de photostabilisation vis-à-vis du rayonnement UV d'au moins filtre UV-B du type ester de l'acide cinnamique en présence d'un filtre UV-A du type dérivé du dibenzoylméthane consistant à associer audit mélange de filtres au moins une s-triazine possédant deux groupes para-aminobenzalmalonate encombrés et un groupe para-aminobenzoate de formule (I) particulière.

La présente invention est relative également à l'utilisation dudit composé s-triazine de dans une composition comprenant dans un support cosmétiquement acceptable, l'association d'un filtre UV-B du type ester de l'acide cinnamique, d'un filtre UV-A du type dérivé de dibenzoylméthane dans le but de d'améliorer l'efficacité de ladite composition vis-à-vis des rayons UV-B ainsi que de maintenir l'équilibre de protection UVA-UVB durant l'exposition solaire.

## Description

La présente invention est relative à une composition en particulier une composition cosmétique à usage topique contenant l'association d'un filtre UV-B du type ester de l'acide cinnamique, d'un filtre UV-A du type dérivé de dibenzoylméthane et d'une s-triazine possédant deux groupes para-aminobenzalmalonate encombrés et un groupe para-aminobenzoate de formule (I) particulière.

Elle concerne également un procédé de photostabilisation vis-à-vis du rayonnement UV d'au moins filtre UV-B du type ester de l'acide cinnamique en présence d'un filtre UV-A du type dérivé du dibenzoylméthane consistant à associer audit mélange de filtres au moins une s-triazine possédant deux groupes para-aminobenzalmalonate encombrés et un groupe para-aminobenzoate de formule (I) particulière.

La présente invention est relative également à l'utilisation dudit composé s-triazine de dans une composition comprenant dans un support cosmétiquement acceptable, l'association d'un filtre UV-B du type ester de l'acide cinnamique, d'un filtre UV-A du type dérivé de dibenzoylméthane dans le but de d'améliorer l'efficacité de ladite composition vis-à-vis des rayons UV-B ainsi que de maintenir l'équilibre de protection UVA-UVB durant l'exposition solaire.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Dans le but d'assurer une protection de la peau et des matières kératiniques contre le rayonnement UV, on utilise généralement des compositions antisolaires comprenant des filtres organiques, actifs dans l'UV-A et actifs dans l'UV-B. La majorité de ces filtres est liposoluble.

A cet égard, une famille de filtres UV-B particulièrement intéressante est actuellement constituée par les esters de l'acide cinnamique et notamment le para-méthoxycinnamate d'éthyl-2-hexyle, qui présentent en effet un fort pouvoir d'absorption intrinsèque. Ces dérivés esters de l'acide cinnamique, qui sont maintenant des produits bien connus en soi à titre de filtres actifs dans les UV-B, sont notamment décrits dans la demande de brevet français FR-A-2315908 ; le para-méthoxycinnamate d'éthyl-2-hexyle est par ailleurs actuellement proposé à la vente sous la dénomination commerciale de « PARSOL MCX » par la Société DSM.

A cet égard également, une famille de filtres UV-A particulièrement intéressante est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de « PARSOL 1789 » par la Société DSM.

L'association de ces deux familles de filtres est extrêmement intéressante pour construire un système de filtration large et équilibré sur l'ensemble du spectre UV.

Malheureusement, il se trouve qu'à la fois les esters de l'acide cinnamique et les dérivés du dibenzoylméthane sont des produits relativement sensibles au rayonnement ultraviolet, c'est-à-dire, plus précisément, qu'ils présentent une fâcheuse tendance à se dégrader plus ou moins rapidement sous l'action de ce dernier.

De plus il se trouve que le fait d'associer un dérivé de dibenzoylméthane à un dérivé ester de l'acide cinnamique donne une instabilité accrue dudit ester de l'acide cinnamique.

Les deux types de filtres étant instables par eux-mêmes et en association , les quantités perdues ne permettent plus d'assurer une bonne couverture de la bande UV et on peut ainsi déséquilibrer le profil de filtration de la formule et induire ainsi une exposition trop importante de la peau ou des cheveux à une partie du spectre UV. Ainsi ce manque substantiel de stabilité des esters de l'acide cinnamique en présence de dérivés de dibenzoylméthane face au rayonnement UV auquel ils sont destinés à être soumis ne permet pas de garantir une protection stable et constante durant une exposition solaire prolongée.

Dans ces conditions la qualité de protection peut être grandement affectée par, d'une part une baisse globale du niveau de protection et d'autre part, par un important déséquilibre de filtration dans l'UV-B. Or si des applications répétées à intervalles de temps réguliers et rapprochées peuvent corriger la baisse du niveau de protection, aucune intervention ne peut compenser le déséquilibre subi par le profil de filtration de la formule.

La demanderesse a découvert de manière surprenante une nouvelle famille de dérivés de s-triazine de formule (I) que l'on définira plus loin en détail possédant deux substituants choisis parmi des groupes para-aminobenzalmalonate et para-aminobenzalmalonamide encombrés et un substituant aminobenzoate ou aminobenzamide permettaient d'améliorer de manière substantielle la stabilité photochimique (ou photostabilité) du mélange des dérivés de l'acide cinnamique et des dérivés de dibenzoylméthane, assurant ainsi tout au long de l'exposition solaire le même équilibre UVB-UVA.

Cette découverte est à la base de la présente invention.

Ainsi, conformément à l'un des objets de la présente invention, il est maintenant proposé une composition comprenant dans un support cosmétiquement acceptable au moins un système filtrant UV, caractérisée par le fait qu'elle comprend au moins :
(a) au moins un filtre UV-B du type dérivé ester de l'acide cinnamique
(b) au moins un filtre UV-A du type dérivé du dibenzoylméthane et
(c) au moins un composé s-triazine de formule (I) dont on donnera la définition ci-après.

Un autre objet de l'invention concerne également un procédé pour améliorer la stabilité chimique vis-à-vis du rayonnement UV d'au moins un filtre UV-B du type dérivé du type dérivé ester de l'acide cinnamique en présence d'un filtre UV-A du type dérivé de dibenzoylméthane consistant à associer à ce mélange de filtres UV au moins un composé s-triazine de formule (I) dont on donnera la définition ci-après.

La présente invention a également enfin pour objet l'utilisation d'un composé s-triazine de formule (I) dont on donnera la définition ci-après, dans une composition comprenant dans un support cosmétiquement acceptable, au moins un filtre UV-B du type dérivé ester de l'acide cinnamique, au moins un dérivé du dibenzoylméthane dans le but de d'améliorer la stabilité chimique vis-à-vis du rayonnement UV dudit filtre UV-B.

La présente invention a également enfin pour objet l'utilisation d'un composé s-triazine de formule (I) dont on donnera la définition ci-après, dans une composition comprenant dans un support cosmétiquement acceptable, au moins un filtre UV-B du type dérivé ester de l'acide cinnamique, au moins un dérivé du dibenzoylméthane dans le but de d'améliorer dans le but de d'améliorer l'efficacité de ladite composition vis-à-vis des rayons UV-B.

La présente invention a également enfin pour objet l'utilisation d'un composé s-triazine de formule (I) dont on donnera la définition ci-après, dans une composition comprenant dans un support cosmétiquement acceptable, au moins un filtre UV-B du type dérivé ester de l'acide cinnamique, au moins un filtre UV-A dérivé du dibenzoylméthane dans le but de maintenir l'équilibre de protection UVA-UVB durant l'exposition solaire.

D'autres caractéristiques, aspects et avantages de l'invention apparaîtront à la lecture de la description détaillée qui va suivre.

Dans la suite de la présente description, on entend par « système filtrant les radiations UV » par un agent filtrant les radiations UV constitué soit d'un composé organique ou minéral unique filtrant les radiations UV soit un mélange de plusieurs composés organiques ou minéraux filtrant les radiations UV, par exemple mélange comprenant un filtre UVA et un filtre UVB.

Par « cosmétiquement acceptable », on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Les esters de l'acide cinnamique conformes à l'invention sont choisis de préférence parmi ceux répondant à la formule (A) suivante : 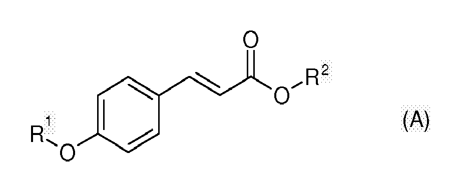 dans laquelle :
R¹, R² sont, indépendamment l'un de l'autre, un radical alkyle en C₁-C₂₄, linéaire ou ramifié et plus particulièrement , un radical alkyle en C₁-C₈, linéaire ou ramifié, tel méthyle, éthyle, propyle, isobutyle, butyle, sec. butyle, isobutyle, pentyle, néopentyle, hexyle et éthyl-2-hexyle.

Parmi les dérivés esters de l'acide cinnamique de formule (A), on peut citer particulièrement
- Ethylhexyl Methoxycinnamate ou Cinoxate vendu notamment sous le nom commercial « PARSOL MCX » par DSM de formule suivante :

- Isopropyl Methoxy cinnamate,
- Isoamyl Methoxy cinnamate vendu sous le nom commercial « NEO HELIOPAN E 1000 » par SYMRISE,
- Diisopropyl Methylcinnamate.

L'ester ou les esters de l'acide cinnamique peuvent être présents dans les compositions conformes à l'invention à des teneurs qui varient de préférence de 0,01 à 20% en poids et plus préférentiellement de 0,1 à 10% en poids et encore plus préférentiellement de 0,1 à 8% en poids par rapport au poids total de la composition.

Parmi les dérivés du dibenzoylméthane, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on utilisera en particulier le 4-isopropyl-dibenzoylméthane, vendu sous la dénomination de "EUSOLEX 8020" par la Société MERCK, et répondant à la formule suivante :

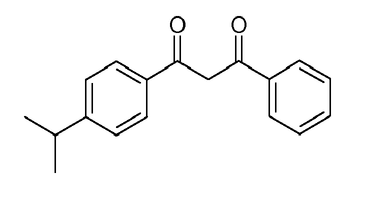

On préfère tout particulièrement mettre en oeuvre le 4-(ter.-butyl) 4'-méthoxy dibenzoylméthane ou Butyl Methoxy Dibenzoylmethane, proposé à la vente sous la dénomination commerciale de "PARSOL 1789" par la Société DSM ; ce filtre répond à la formule suivante :

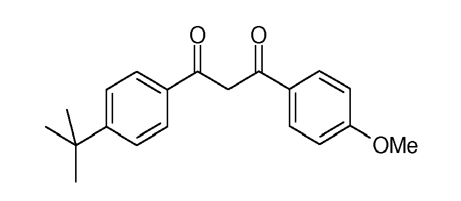

Le ou les dérivés du dibenzoylméthane peuvent être présents dans les compositions conformes à l'invention à des teneurs qui varient de préférence de 0,01 à 20% en poids et plus préférentiellement de 0,1 à 10% en poids et encore plus préférentiellement de 0,1 à 6% en poids par rapport au poids total de la composition.

Les composés s-triazine conformes à la présente invention répondent à la formule générale (I) suivante : 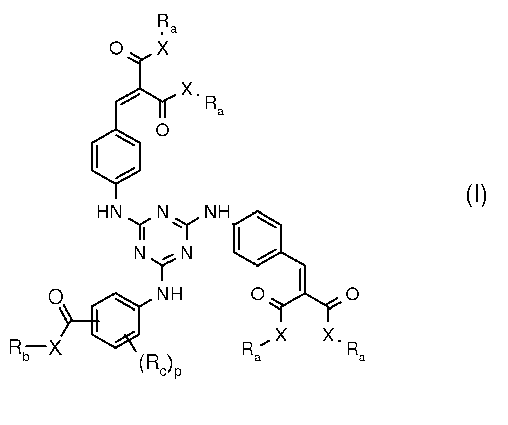 dans laquelle :
X, identiques ou différents, désignent -O- ou -NR₆-
Rₐ, identiques ou différents, désignent un groupe de formule (II): 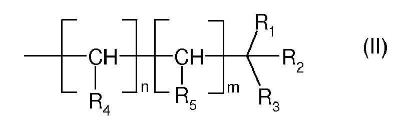
dans laquelle :
R₁ et R₂, identiques ou différents représentent un groupe alkyle en C₁-C₈, linéaire ou ramifié,
R₁ et R₂ peuvent former un cycle en C₅-C₈, éventuellement substitué par 1, 2 ou 3 groupements alkyle(s) en C₁-C₄, linéaire(s) ou ramifié(s) ;
R₃, R₄ et R₅, identiques ou différents représentent un atome d'hydrogène ou un groupe alkyle en C₁-C₄, linéaire ou ramifié ;
n vaut 0 ou 1 ;
m vaut 0 ou 1 ;
sous réserve que :
(i) lorsque n = 1 et R₄ désigne l'hydrogène alors m est égal à 0 et R₃ est différent d'hydrogène ;
(ii) lorsque R₁ et R₂ forment un cycle en C₅-C₈ alors la somme n+m est différente de 2 ; R₆ représente l'hydrogène ou un groupe alkyle en C₁-C₈,
R_{b} désigne un groupe alkyle en C₁-C₂₀, linéaire ou ramifié et éventuellement insaturé, un groupe cycloalkyle en C₅-C₁₂, éventuellement substitué par 1 à 3 radicaux alkyles en C₁-C₄, linéaires ou ramifiés, le groupe -(CH₂CHR₇-O)_{q}R₈ ou le groupe -CH₂-CH(OH)-CH₂-O-R₈,
R₇ représente l'hydrogène ou méthyle,
R₈ représente l'hydrogène ou un groupe alkyle en C₁-C₈,
q = 1-20,
le groupement COXR_{b} peut être en position ortho, méta ou para du groupement amino,
R_{c} désigne un radical alkyle en C₁-C₂₀, linéaire ou ramifié, saturé ou insaturé, le radical OH, un radical alcoxy en C₁-C₂₀, linéaire ou ramifié,
p est égal à 0, 1 ou 2.

Dans la formule (I) ci-dessus, les radicaux alkyles peuvent être choisis notamment au sein des radicaux méthyle, éthyle, n-propyle, isopropyle, n-butyle, isobutyle, tert-butyle, n-amyle, isoamyle, néopentyle, n-hexyle, n-heptyle, n-octyle, éthyl-2 hexyle et tert-octyle. Le radical alkyle particulièrement préféré est le radical méthyle.

Les radicaux cycloalkyles peuvent être choisis notamment au sein des radicaux cyclopentyle, cyclohexyle et cycloheptyle. Le radical cycloalkyle particulièrement préféré est le radical cyclohexyle. Ces radicaux peuvent être substitués par des radicaux alkyle en C₁-C₄ choisis de préférence parmi méthyle, isopropyle et tert-butyle.

Parmi les composés de formule (I) préférés, on citera ceux pour lesquels les deux conditions suivantes sont réunies :
(a) n=m=0 et
(b) R₁, R₂, R₃ désignent un alkyle en C₁-C₄ et plus particulièrement méthyle ou bien R₃ désigne hydrogène et R₁ et R₂ forment un cycle en C₅-C₈ éventuellement substitué par 1 ou deux radicaux alkyle et plus particulièrement cyclohexyle.

Parmi les composés de formule (I) préférés, on citera également ceux pour lesquels les deux conditions suivantes sont réunies :
(a) n=1 et R₄ désigne un alkyle en particulier méthyle ou m = 1 et R₅ désigne un alkyle en particulier méthyle et
(b) R₁ et R₂ désignent un alkyle en C₁-C₄ et plus particulièrement méthyle.

Parmi les composés de formule (I) plus particulièrement préférés, on citera ceux choisis parmi les composés de formules (1) à (10) suivantes :

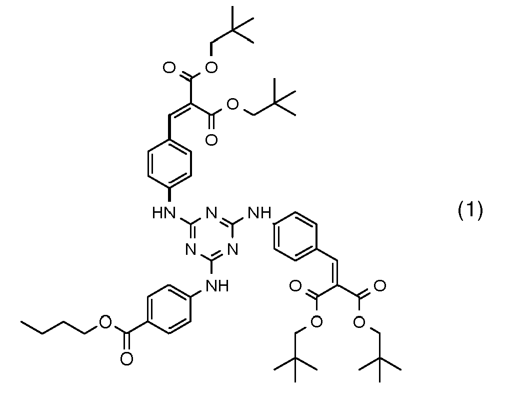

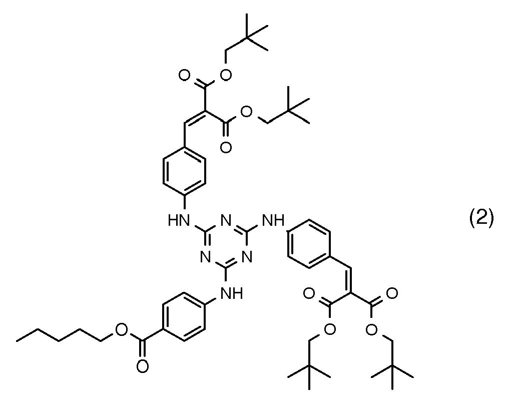

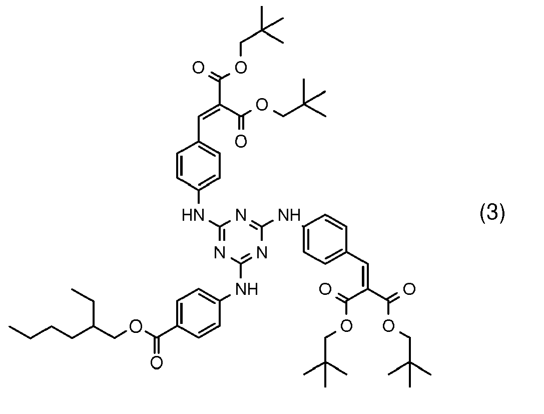

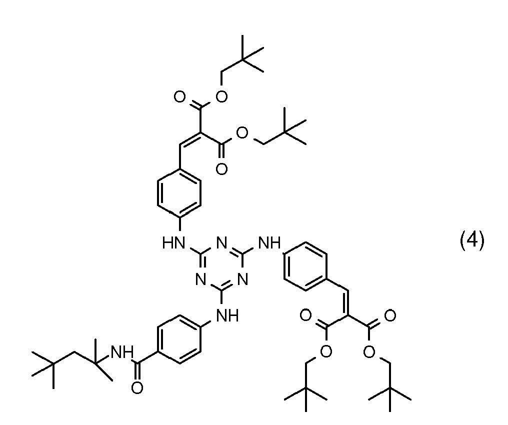

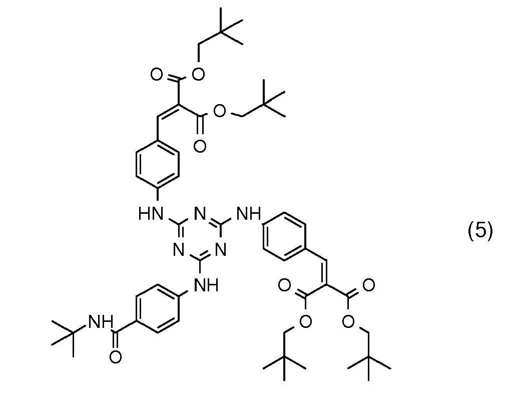

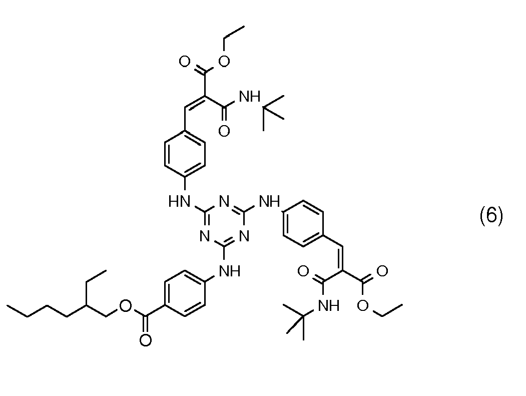

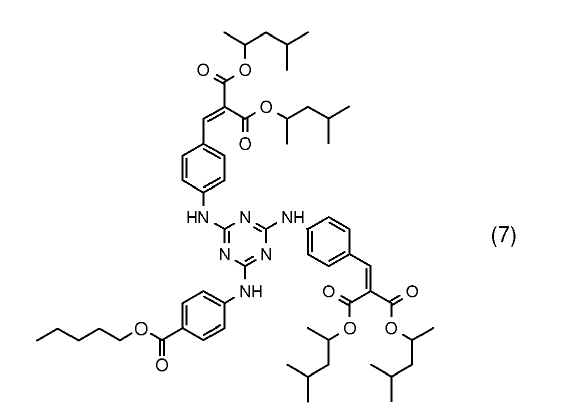

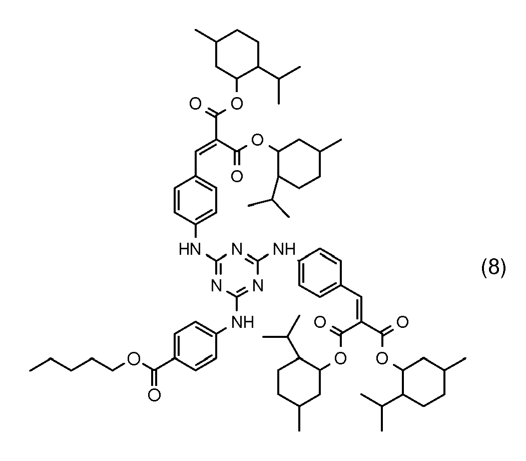

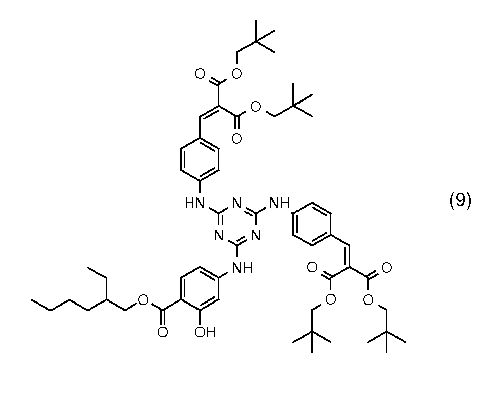

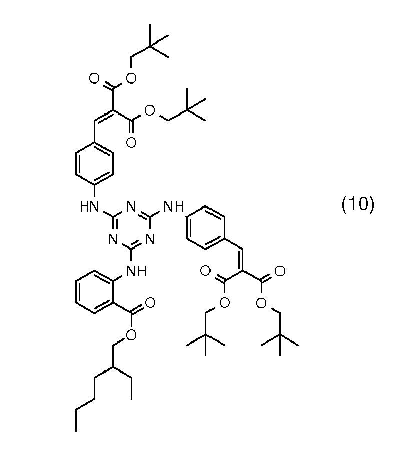

Parmi ces composés, on utilisera plus particulièrement le 2,4-bis-(4'-amino benzalmalonate de di-néopentyle)-6-(4"-amino benzoate de butyle)-s-triazine de formule (1).

Les dérivés de formule (I) peuvent être obtenus selon le schéma (A) suivant : 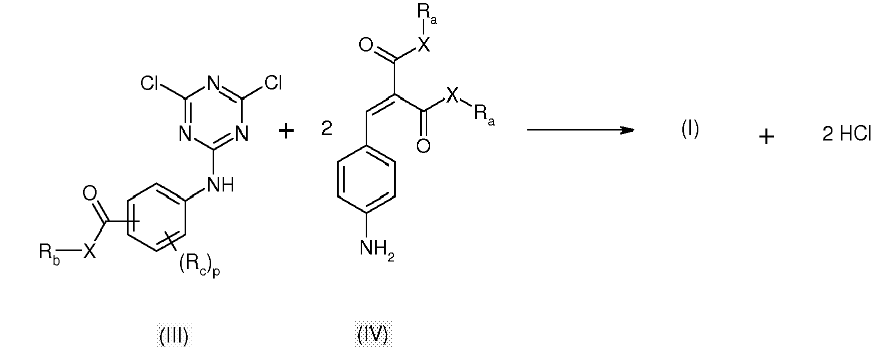 dans lequel Rₐ, X, R_{b}, R_{c} et p ont la définition de la formule (I) ci-dessus.

Les réactions ci-dessus peuvent être effectuées éventuellement en présence d'un solvant (par exemple : toluène, xylène ou dichloro-1,2-éthane), à une température comprise entre 0 °C et 250 °C, plus particulièrement entre 5 °C et 150 °C. Elles peuvent être également réalisées en microondes en présence ou non d'un solvant (par exemple : toluène, xylène ou dichloro-1,2-éthane) ou en présence ou non de 10% de graphite, à une température de 50 à 150°C, à une puissance de 50-150 Watts pendant une durée de 10 à 30 minutes.

Les composés de formule (III) peuvent être préparés selon des méthodes connues décrites par exemple dans la demande EP 0 507 691 de la Demanderesse.

Les composés de formule (I) sont généralement présents dans la composition de l'invention dans des proportions comprises entre 0,01 % et 20 % en poids, de préférence entre 0,1 % et 10 % en poids, par rapport au poids total de la composition.

Les compositions conformes à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Les filtres organiques complémentaires sont notamment choisis parmi les anthranilates ; les dérivés salicyliques, les dérivés du camphre ; les dérivés de triazine autres que celles de l'invention tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate autres que ceux de l'invention ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCl :
Dérivés de l'acide para-aminobenzoique :
   PABA,
   Ethyl PABA,
   Ethyl Dihydroxypropyl PABA,
   Ethylhexyl Diméthyl PABA vendu notamment sous le nom « ESCALOL 507 » par ISP, Glyceryl PABA,
   PEG-25 PABA vendu sous le nom « UVINUL P25 » par BASF,
Dérivés salicyliques :
   Homosalate vendu sous le nom « Eusolex HMS » par Rona/EM Industries,
   Ethylhexyl Salicylate vendu sous le nom « NEO HELIOPAN OS » par HAARMANN et REIMER,
   Dipropyleneglycol Salicylate vendu sous le nom « DIPSAL » par SCHER,
   TEA Salicylate, vendu sous le nom « NEO HELIOPAN TS » par HAARMANN et REIMER,
Dérivés de β,β'-diphénylacrylate:
   Octocrylene vendu notamment sous le nom commercial « UVINUL N539 » par BASF,
   Etocrylene, vendu notamment sous le nom commercial « UVINUL N35 » par BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF,
   Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial« UVINUL DS-49» par BASF,
   Benzophenone-12
   Diethylamino Hydroxybenzoyl Hexyl Benzoate vendu sous le nom commercial « UVINUL A PLUS» par BASF,
Dérivés du benzylidène camphre :
   3-Benzylidene camphor fabriqué sous le nom « MEXORYL SD» par CHIMEX,
   4-Methylbenzylidene camphor vendu sous le nom « EUSOLEX 6300 » par MERCK ,
   Benzylidene Camphor Sulfonic Acid fabriqué sous le nom « MEXORYL SL» par CHIMEX,
   Camphor Benzalkonium Methosulfate fabriqué sous le nom « MEXORYL SO » par CHIMEX,
   Terephthalylidene Dicamphor Sulfonic Acid fabriqué sous le nom « MEXORYL SX » par CHIMEX,
   Polyacrylamidomethyl Benzylidene Camphor fabriqué sous le nom « MEXORYL SW » par CHIMEX,
Dérivés du phenyl benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate vendu sous le nom commercial commercial « NEO HELIOPAN AP » par HAARMANN et REIMER,
Dérivés de la triazine :
   Bis-Ethylhexyloxyphenol Methoxyphenyl Triazine vendu sous le nom commercial «TINOSORB S » par CIBA GEIGY,
   Ethylhexyl triazone vendu notamment sous le nom commercial «UVINUL T150 » par BASF,
   Diethylhexyl Butamido Triazone vendu sous le nom commercial « UVASORB HEB » par SIGMA 3V,
Dérivés du phenyl benzotriazole :
   Drometrizole Trisiloxane vendu sous le nom « Silatrizole » par RHODIA CHIMIE Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliques :
   Menthyl anthranilate vendu sous le nom commercial commercial « NEO HELIOPAN MA » par HAARMANN et REIMER,
Dérivés d'imidazolines :
   Ethylhexyl Dimethoxybenzylidene Dioxoimidazoline Propionate,
Dérivés du benzalmalonate :
   Polyorganosiloxanes à fonction benzalmalonate tels que le Polysilicone-15 vendu sous la dénomination commerciale « PARSOL SLX » par HOFFMANN LA ROCHE
Dérivés de 4,4-diarylbutadiène :
   - 1,1-dicarboxy (2,2'-diméthyl-propyl)-4,4-diphénylbutadiène
   et leurs mélanges.

Les filtres UV organiques complémentaires préférentiels sont choisis parmi
Ethylhexyl Salicylate,
Homosalate,
Octocrylene,
Phenylbenzimidazole Sulfonic Acid,
Disodium Phenyl Dibenzimidazole Tetra-sulfonate,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
et leurs mélanges.

Les agents photoprotecteurs complémentaires inorganiques sont choisis parmi des pigments et plus préférentiellement encore des pigments ayant une taille moyenne des particules primaires généralement entre 5 nm et 100 nm, de préférence entre 10 nm et 50 nm) d'oxydes métalliques traités ou non comme par exemple des pigments d'oxyde de titane (amorphe ou cristallisé sous forme rutile et/ou anatase), de fer, de zinc, de zirconium ou de cérium.

Les pigments traités sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p 53-64, tels que des aminoacides, de la cire d'abeille, des acides gras, des alcools gras, des tensio-actifs anioniques, des lécithines, des sels de sodium, potassium, zinc, fer, ou aluminium d'acides gras, des alcoxydes métalliques (de titane ou d'aluminium), du polyéthylène, des silicones, des protéines (collagène, élastine) des alcanolamines, des oxydes de silicium, des oxydes métalliques, de l'hexamétaphosphate de sodium, de l'alumine ou de la glycérine.

Les pigments traités peuvent être plus particulièrement des oxydes de titane traités par :
- la silice et l'alumine tels que les produits « Microtitanium Dioxide MT 500 SA » et « Microtitanium Dioxide MT 100 SA » de la société TAYCA, et les produits « Tioveil Fin », « Tioveil OP », « Tioveil MOTG » et « Tioveil IPM » de la société TIOXIDE,
- l'alumine et le stéarate d'aluminium tels que le produit « Microtitanium Dioxide MT 100 T » de la société TAYCA,
- l'alumine et le laurate d'aluminium tels que le produit « Microtitanium Dioxide MT 100 S » de la société TAYCA,
- des oxydes de fer et le stéarate de fer tels que le produit « Microtitanium Dioxide MT 100 F » de la société TAYCA,
- la silice, l'alumine et la silicone tels que les produits « Microtitanium Dioxide MT 100 SAS », « Microtitanium Dioxide MT 600 SAS » et « Microtitanium Dioxide MT 500 SAS » de la société TAYCA,
- l'hexamétaphosphate de sodium tels que le produit « Microtitanium Dioxide MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212» de la société KEMIRA,
- l'alumine et la silicone tels que le produit « UVT-M262» de la société KEMIRA.

D'autres pigments d'oxyde de titane traités avec une silicone sont de préférence le TiO₂ traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 Cardre UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple
- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple
- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C₁₂-C₁₅) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% d'oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C₁₂-C₁₅ avec polycondensat d'acide hydroxystéarique).

Les pigments d'oxyde de cérium non enrobé est vendu sous la dénomination "COLLOIDAL CERIUM OXIDE" par la société RHONE POULENC.
Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261 " vendu par la société KEMIRA ou enrobé d'alumine, de silice et de glycérine tel que le produit "M 211" vendu par la société KEMIRA.

Les pigments peuvent être introduits dans les compositions selon l'invention tels quels ou sous forme de pâte pigmentaire, c'est-à-dire en mélange avec un dispersant, comme décrit par exemple dans le document GB-A-2206339.

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les carbopols (carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide) ; les dérivés cellulosiques tels que l'hydroxyéthylcellulose ; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Parmi les actifs, on peut citer :
- les agents anti-pollution et/ou agent anti-radicalaire ;
- les agents dépigmentants et/ou des agents pro-pigmentants ;
- les agents anti-glycation ;
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants ;
- les agents tenseurs.
- les agents desquamants ;
- les agents hydratants ;
- les agents anti-inflammatoires ;
- les agents agissant sur le métabolisme énergétique des cellules.
- les agents répulsifs contre les insectes
- les antagonistes de substances P ou de CRGP.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile. Elles peuvent se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostearate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin et des produits de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Des exemples concrets, mais nullement limitatifs, illustrant l'invention, vont maintenant être donnés.

### EXEMPLE 1 : Préparation du 2,4-bis-(4'-aminobenzalmalonate de di-néopentyle)-6-(4 "-aminobenzoate de butyle)-s-triazine de formule (1) :

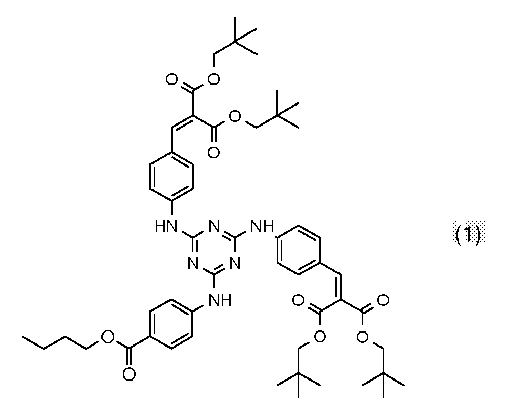

### Première étape : préparation du 2,4-dichloro-6-(4'-amino benzoate de butyle)-s-triazine

Dans un réacteur, on solubilise à 0-5°C le chlorure de cyanuryle (20,7 g, 0,112 mole) dans 250 ml d'acétone. On y ajoute goutte à goutte à 0-5°C en 1 heure une solution de para aminobenzoate de butyle (21,7 g, 0,112 mole) dissout dans 70 ml d'acétone. Ensuite, on y ajoute du bicarbonate de sodium (9,4 g, 0,112 mole) dissout dans 70 ml d'eau. Le mélange hétérogène est laissé 2 heures à une température de 0-5°C. Le précipité formé est filtré, puis lavé à l'eau et à l'acétone. Après séchage sous vide, on obtient 37,2 g (Rendement 97%) du 2,4-dichloro-6-(4'-amino benzoate de butyle)-s-triazine sous forme d'une poudre blanche :
UV (Ethanol/DMSO) : λmax = 298 nm , E1% = 940,
et utilisée telle quelle dans l'étape suivante.

### Deuxième étape : préparation du dérivé de l'exemple 1 :

Sous barbotage d'azote, un mélange du produit précédent (7,41 g, 0,0213 mole) et de para-amino benzalmalonate de dinéopentyle (14,66 g, 0,0422 mole) en suspension dans 60 ml de toluène est chauffé au reflux pendant 7 heures 30 minutes. On refroidit et ajoute du dichlorométhane. La phase organique est lavée avec une solution saturée de bicarbonate de sodium puis à l'eau. La phase organique est séchée puis concentrée sous pression réduite. L'huile orangée obtenue (17,8 g) est soumise à une séparation sur colonne de silice (éluant : Heptane/EtOAc 85 :15). On récupère des fractions propres sous forme de paillettes jaunes pâles du dérivé de l'exemple 1 (8,72 g, Rendement 43%) :
UV (Ethanol) : λ = 370 nm , E1% = 623; λmax = 347 nm , E1% = 847; λ = 300 nm , E1% = 432.

### EXEMPLE 2 : Préparation du 2,4-bis-(4'-aminobenzalmalonate de di-néopentyle)-6-(4 "-aminobenzoate d'amyle)-s-triazine de formule (2) :

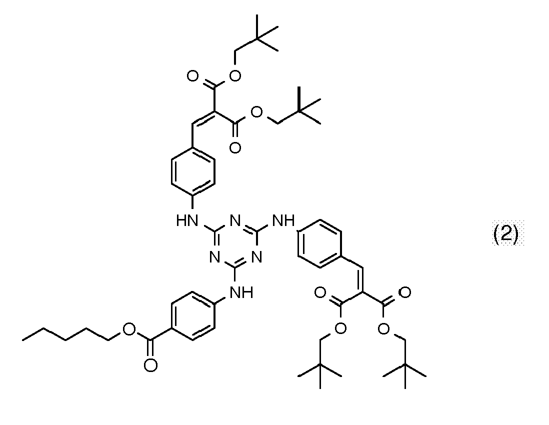

### Première étape : préparation du 2,4-dichloro-6-(4'-amino benzoate d'amyle)-s-triazine :

Dans un réacteur on solubilise à 10°C le chlorure de cyanuryle (14,7 g, 0,0796 mole) dans 200 ml de dioxanne. On y ajoute simultanément goutte à goutte à 10°C en 1 heure une solution de para aminobenzoate d'amyle (16,5 g, 0,0796 mole) dissout dans 60 ml de dioxanne et une solution de carbonate de potassium (5,5 g, 0,0398 mole) dissout dans 30 ml d'eau. Le mélange hétérogène est laissé 2 heures à une température de 10°C. On ajoute environ 300 ml d'eau et le précipité formé est filtré, puis lavé à l'eau. Après séchage sous vide, on obtient 26,4 g (Rendement 93%) du 2,4-dichloro-6-(4'-amino benzoate d'amyle)-s-triazine sous forme d'une poudre blanche et utilisée telle quelle dans l'étape suivante.

### Deuxième étape : préparation du dérivé de l'exemple 2 :

Dans un micro ondes CEM Discover, on laisse le mélange intimement mélangé du produit précédent (0,103 g, 0,29x10-3 mole), de para-amino benzalmalonate de dinéopentyle (0,2 g, 0,58x10-3 mole) et de bicarbonate de sodium (0,049g, 0,58x10-3 mole) pendant 10 minutes à une température de 150°C et sous une puissance de 150 Watt. On extrait le solide amorphe formé au dichlorométhane. La phase organique est lavée 3 fois à l'eau et est séchée puis concentrée sous pression réduite. L'huile jaune obtenue est soumise à une séparation sur colonne de silice (éluant : Heptane/EtOAc 80 :20). On récupère des fractions propres sous forme d'une pâte jaune pâle du dérivé de l'exemple 2 (36 mg, Rendement 15%) :
UV (Ethanol) : λ = 375 nm , E1% = 610; λmax = 347 nm , E1% = 834; λ = 300 nm , E1%=425.

### EXEMPLE 3 : Préparation du 2,4-bis-(4'-aminobenzalmalonate de di-néopentyle)-6-(4"-aminobenzoate d'éthyl-2-hexyle)-s-triazine de formule (3) :

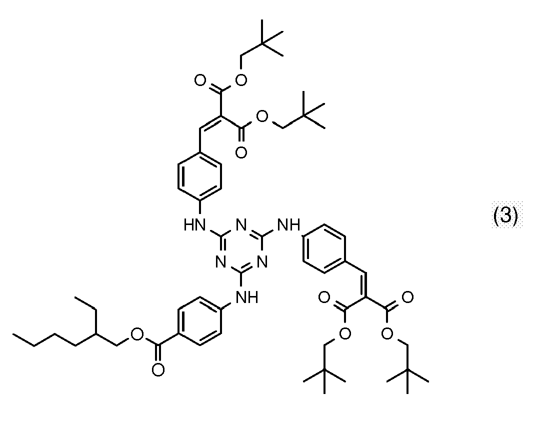

### Première étape : préparation du 2,4-dichloro-6-(4'-amino benzoate d'éthyle-2-hexyle)-s-triazine :

Dans un réacteur, on solubilise à 0-5°C le chlorure de cyanuryle (18,4 g, 0,1 mole) dans 150 ml d'acétone. On y ajoute du bicarbonate de sodium (10,6 g, 0,1 mole) puis on ajoute goutte à goutte à une température inférieure à 10°C en 10 minutes une solution de para aminobenzoate d'éthyle-2-hexyle (24,9 g, 0,1 mole) dissout dans 150 ml d'acétone. Ensuite le mélange hétérogène est laissé 3 heures à la température du labo. On verse 500 ml d'eau. Le précipité formé est filtré, puis lavé à l'eau. Après séchage sous vide, on obtient 38 g d'un solide blanc cassé. Après recristallisation de ce solide dans le dichloro-1,2-éthane, on obtient 25,2g (Rendement 63%) du 2,4-dichloro-6-(4'-amino benzoate d'éthyle-2-hexyle)-s-triazine sous forme d'une poudre blanche :
UV (Ethanol/DMSO) : λmax = 291 nm , E1% = 732,
et utilisée telle quelle dans l'étape suivante.

### Deuxième étape : préparation du dérivé de l'exemple 3 :

Sous barbotage d'azote, un mélange du produit précédent (1,14 g, 2,87x10-3 mole) et de para-amino benzalmalonate de dinéopentyle (2,2 g, 6,33x10-3 mole) en suspension dans 35 ml de toluène est chauffé au reflux pendant 10 heures 30 minutes. On refroidit et ajoute du dichlorométhane. La phase organique est lavée avec une solution saturée de bicarbonate de sodium puis à l'eau. La phase organique est séchée puis concentrée sous pression réduite. L'huile orangée obtenue (2,6 g) est soumise à une séparation sur colonne de silice (éluant : Heptane/EtOAc 85 :15). On récupère des fractions propres sous forme de paillettes jaunes pâles du dérivé de l'exemple 3 (1,17 g, Rendement 40%) :
UV (Ethanol) :
   λ = 370 nm, E1% = 575; λmax = 342 nm, E1% = 880; λ = 300 nm , E1% = 448.

### EXEMPLE 4 : Préparation du 2,4-bis-(4'-aminobenzalmalonate de dinéopentyle)-6-(4"-aminobenzamide de tert-octyle)-s-triazine de formule (4) :

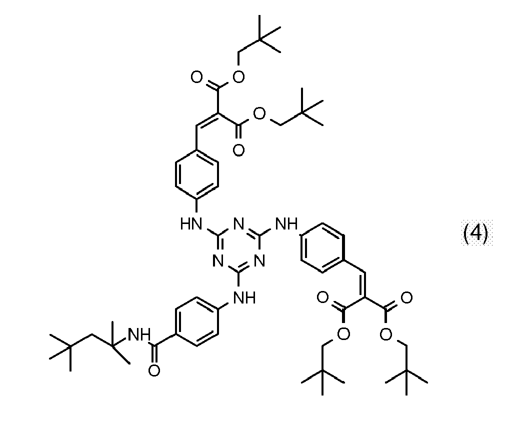

### Première étape : préparation du 4-nitro-N-(tert-octyl) benzamide :

Dans un réacteur, on introduit de la tert-octyl amine (51,7 g, 0,4 mole) et de la triéthylamine (61,2 ml, 0,44 mole) dans 260 ml de dichloroéthane. On chauffe à 70°C puis on ajouteen 50 minutes le 4-nitrobenzoyl chloride (77,9 g, 0,42 mole) par petites portions. On chauffe au reflux pendant 4 heures.. On verse le mélange sur de l'eau glacée ; on extrait au dichlorométhane, sèche et évapore le solvant. Le précipité beige obtenu est recristallisé dans un mélange d'éther isopropylique et d'éthanol (rapport 10 :1). Après séchage sous vide, on obtient 84,6 g (Rendement 76%) du 4-nitro-N-(tert-octyl) benzamide sous forme d'une poudre blanc cassé et utilisée telle quelle dans l'étape suivante.

### Deuxième étape : préparation du 4-amino-N-(tert-octyl) benzamide :

Dans un hydrogénateur de 500 ml, du 4-nitro-N-(tert-octyl) benzamide (30 g, 0,108 mole) dissout dans 200 ml d'acétate d'éthyle est hydrogéné en présence de 4,8 g de palladium à 10% sur charbon à 50% d'eau comme catalyseur (pression d'hydrogène : 8-10 bars) à une température de 70-75°C pendant 1 heure et 15 minutes. Après filtration, concentration du solvant et séchage sous vide, on obtient 20,4 g (Rendement : 76%) de 4-amino-N-(tert-octyl) benzamide sous forme d'une poudre jaune clair et utilisée telle quelle dans l'étape suivante.

### Troisième étape : préparation du N-(tert-octyl)-4-[(4,6-dichloro-1,3,5-triazin-2-yl)amino] benzamide :

Dans un réacteur, on solubilise à 10°C le chlorure de cyanuryle (3,7 g, 0,0201 mole) dans 70 ml de dioxanne. On y ajoute simultanément goutte à goutte à 10°C en 1 heure une solution du produit de l'étape précédente (5 g, 0,0201 mole) dissout dans 100 ml de dioxanne et une solution de carbonate de potassium (1,4 g, 0,03 mole) dissout dans 20 ml d'eau. Le mélange hétérogène est laissé 2 heures à une température de 10°C. On ajoute environ 300 ml d'eau et le précipité formé est filtré, puis lavé à l'eau. Après séchage sous vide, on obtient 7,4 g (Rendement 93%) du N-(tert-octyl)-4-[(4,6-dichloro-1,3,5-triazin-2-yl)amino] benzamide sous forme d'une poudre blanche et utilisée telle quelle dans l'étape suivante.

### Quatrième étape : préparation du dérivé de l'exemple 4 :

Dans un micro ondes CEM Discover, on laisse le mélange intimement mélangé du produit de l'étape précédente (0,29 g, 0,732x10-3 mole), de para-amino benzalmalonate de dinéopentyle (0,5 g, 1,44x10-3 mole) et de bicarbonate de sodium (0,14 g, 1,44x10-3 mole) pendant 4 minutes à une température de 60°C et sous une puissance de 300 Watt puis pendant 15 minutes à une température de 110°C. On extrait le solide amorphe formé au dichlorométhane. La phase organique est lavée 3 fois à l'eau et est séchée puis concentrée sous pression réduite. L'huile orangée obtenue est soumise à une séparation sur colonne de silice (éluant : Heptane/EtOAc 75 :25). On récupère des fractions propres sous forme d'une huile jaune pâle qui se solidifie pour donner le dérivé de l'exemple 4 (0,6 g, Rendement 86%) sous forme d'une poudre jaune pâle :
UV (Ethanol) :
   λ max = 351 nm , E1% = 763; λmax = 298 nm , E1% = 369.

### EXEMPLE 5 : Préparation du 2,4-bis-(4'-aminobenzalmalonate de di-néopentyle)-6-(4"-aminobenzamide de tert-butyle)-s-triazine de formule (5) :

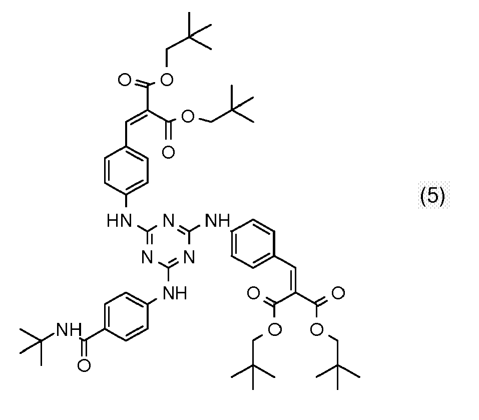

### Première étape : préparation du N-(tert-butyl)-4-nitro benzamide :

Dans un réacteur, on ajoute en 30 minutes à une température de 0-5°C du chlorure de 4-nitrobenzoyle (18,9 g, 0,1 mole) dissout dans 60 ml de chlorure de méthylène à une solution de tert-butylamine (8,3 g, 0,112 mole) et de triéthylamine (15,6 ml, 0,112 mole) dissouts dans 170 ml de dichlorométhane. Le mélange réactionnel est ramené à la température du labo et laissé sous agitation pendant 2 heures. La phase organique est lavée 2 fois à l'eau, séchée. Après élimination du solvant sous pression réduite, le solide obtenu est recristallisé dans l'isopropanol. On obtient 17,1 g (Rendement : 77%) de N-(tert-butyl)-4-nitro benzamide sous forme d'une poudre jaune pâle (Pf 161-2°C) et utilisé tel quel dans l'étape suivante.

### Deuxième étape : préparation de 4-amino-N-(tert-butyl) benzamide :

Dans un hydrogénateur de 1 litre, le produit précédent (17,1 g, 0,077 mole) dissout dans 300 ml d'isopropanol est hydrogéné en présence de 3 g de palladium sur charbon à 5% comme catalyseur (pression d'hydrogène : 7 bars) à une température de 60°C pendant 30 minutes. Après filtration, concentration du solvant et séchage sous vide, on obtient 13,2 g (Rendement : 89%) de 4-amino-N-(tert-butyl) benzamide sous forme d'une poudre gris clair (Pf 123-4°C) et utilisée telle quelle dans l'étape suivante.

### Troisième étape : préparation du N-(tert-butyl)-4-[(4,6-dichloro-1,3,5-triazin-2-yl)amino] benzamide :

Dans un réacteur, on solubilise à 10°C le chlorure de cyanuryle (11,71 g, 0,06 mole) dans 150 ml de dioxanne. On y ajoute simultanément goutte à goutte à 10°C en 1 heure une solution du produit de l'étape précédente (11,53 g, 0,06 mole) dissout dans 60 ml de dioxanne et une solution de carbonate de potassium (6,3 g, 0,03 mole) dissout dans 30 ml d'eau. Le mélange hétérogène est laissé 2 heures à une température de 10°C. On ajoute environ 300 ml d'eau et le précipité formé est filtré, puis lavé à l'eau. Après séchage sous vide, on obtient 18 g (Rendement 88%) du N-(tert-butyl)-4-[(4,6-dichloro-1,3,5-triazin-2-yl)amino] benzamide sous forme d'une poudre blanche (Pf 256-7°C) et utilisée telle quelle dans l'étape suivante.

### Quatrième étape : préparation du dérivé de l'exemple 5 :

Dans un micro ondes CEM Discover, on laisse le mélange intimement mélangé du produit précédent (1,5 g, 4,4x10-3 mole), de para-amino benzalmalonate de dinéopentyle (3 g, 8,8x10-3 mole) et de bicarbonate de sodium (0,37 g, 8,8x10-3 mole) pendant 4 minutes à une température de 60°C et sous une puissance de 300 Watt puis pendant 20 minutes à une température de 150°C. On extrait le solide amorphe formé au dichlorométhane. La phase organique est lavée 3 fois à l'eau et est séchée puis concentrée sous pression réduite. L'huile marron obtenue est soumise à une séparation sur colonne de silice (éluant : Heptane/EtOAc 60 :40). On récupère des fractions propres sous forme d'une poudre jaune pâle du dérivé de l'exemple 5 (0,7 g, Rendement 17%) :
UV (Ethanol) :
   λ = 375 nm , E1% = 420; λmax = 345 nm , E1% = 813; λ = 299 nm , E1% = 420.

### EXEMPLE 6 : Préparation du 2,4-bis-(4'-aminobenzalmalonate de 1,3-diméthylbutyle)-6-(4"-aminobenzoate d'amyle)-s-triazine de formule (6) :

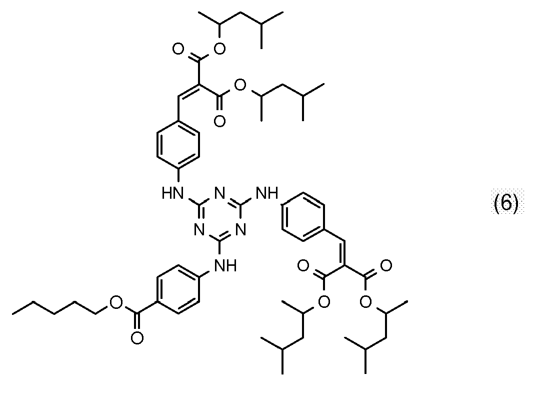

### Première étape : préparation du malonate de 1,3-diméthylbutyle :

Dans un réacteur surmonté par un Dean Stark, on porte au reflux pendant 2 heures l'acide malonique (72,8 g, 0,7 mole) et l'alcool 2-méthyl-4-pentanol (286 g, 2,8 mole) dans 200 ml de toluène en présence de 1,8 ml d'acide sulfurique concentré. L'eau formée est éliminée par azéotropie. La phase organique est lavée 3 fois à l'eau et est séchée sur sulfate de sodium. On filtre et on évapore le solvant. Le produit obtenu distille à 147°C sous 20 hPa. On obtient 160 g (Rendement 79 %) du malonate de 1,3-diméthylbutyle sous forme d'une huile incolore qui est utilisée telle quelle dans l'étape suivante.

### Deuxième étape : préparation du 4-nitrobenzalmalonate de 1,3-diméthylbutyle

Dans un ballon équipé d'un Dean Stark surmonté d'un réfrigérant et sous barbotage d'azote, on place le p-nitro benzaldéhyde (49,9 g, 0,33 mole) et le malonate de 1,3-diméthylbutyle (90 g, 0,33 mole) dans 150 ml de toluène. On y ajoute le catalyseur préparé par avance, l'acide acétique (1,9 ml) et la pipéridine (3,3 ml) en suspension dans 4 ml de toluène. On porte le mélange sous agitation au reflux pendant 7 heures 30 minutes et élimine par l'intermédiaire du Dean Stark l'eau formée. Deux rajouts des mêmes quantités de catalyseur ont été nécessaires. On verse le mélange réactionnel refroidi dans l'eau et extrait au dichlorométhane. La phase organique est lavée à l'eau, puis séchée et concentrée sous pression réduite. L'huile marron rouge obtenue est chromatographiée sur colonne de silice (éluant : Heptane/EtOAc 97:3). On récupère 56,8 g (Rendement 43%) des fractions propres de 4-nitrobenzalmalonate de 1,3-diméthylbutyle sous forme d'une huile jaune et utilisée telle quelle dans l'étape suivante.

### Troisième étape : préparation du 4-aminobenzalmalonate de 1,3-diméthylbutyle

Sous agitation et barbottage d'azote, on disperse le dérivé de l'étape précédente (56,8 g, 0,14 mole) dans 80 ml d'acide acétique. On y ajoute 115 ml d'eau. Le mélange est chauffé à 50 °C. On y ajoute le fer (78,2 g, 1,4 mole) par portions sans dépasser une température de 55°C (temps d'introduction 1 heure). Ensuite, on ajoute goutte à goutte de l'acide acétique (115 ml) sans dépasser une température de 55°C (temps d'introduction 2 heures). On chauffe 1 heure de plus à 55°C. On refroidit, ajoute de l'eau et extrait 2 fois au dichlorométhane. La phase organique est lavée à l'eau, avec une solution saturée de bicarbonate de sodium, à l'eau puis est séchée sur sulfate de sodium. Après concentration sous pression réduite, on obtient une huile marron rouge que l'on purifie par passage sur colonne de silice (éluant : Heptane/EtOAc 85 :15). On le recristallise dans un mélange d' heptane et de dichloro-1,2 éthane. On récupère 22,5 g (Rendement 43%) des fractions propres de 4-aminobenzalmalonate de 1,3-diméthylbutyle sous forme d'une huile orangé et utilisée telle quelle dans l'étape suivante.

### Quatrième étape : préparation du dérivé de l'exemple 6 :

Dans un micro ondes CEM Discover, on laisse le mélange intimement mélangé du produit de l'étape précédente (0,5 g, 1,32x10-3 mole), du produit de la deuxième étape de l'exemple 2 (0,235 g, 0,66x10-3 mole) et de bicarbonate de sodium (0,11 g, 1,32x10-3 mole) pendant 4 minutes à une température de 60°C et sous une puissance de 300 Watt puis pendant 25 minutes à une température de 110°C. On extrait le solide amorphe formé au dichlorométhane. La phase organique est lavée 3 fois à l'eau et est séchée puis concentrée sous pression réduite. L'huile orangée obtenue est soumise à une séparation sur colonne de silice (éluant : Heptane/EtOAc 80 :20). On récupère des fractions propres sous forme d'une huile jaune qui se solidifie pour donner le dérivé de l'exemple 6 (0,4 g, Rendement 57%) sous forme de paillettes jaune clair :
UV (Ethanol) :
   λ = 370 nm, E1% = 500; λmax = 337 nm, E1% = 800; λ = 300 nm, E1% = 411.

### EXEMPLE 7 : Préparation du 2,4-bis-(4'-aminobenzalmalonate de di-menthyle)-6-(4"-aminobenzoate d'amyle)-s-triazine de formule (7) :

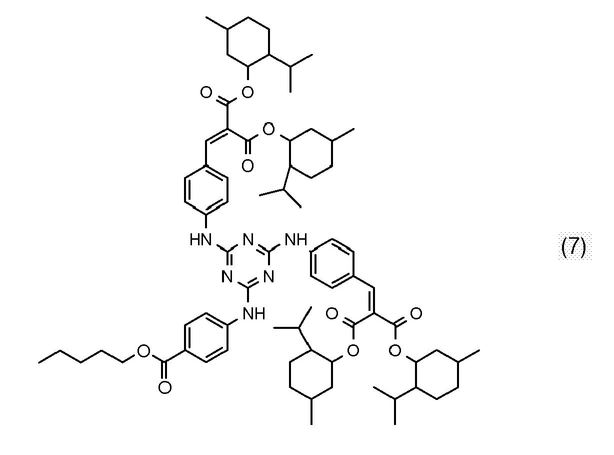

### Première étape : préparation du malonate de di-menthyle :

Dans un réacteur surmonté par un Dean Stark, on porte au reflux pendant 6 heures l'acide malonique (22,2 g, 0,213 mole) et le menthol (70 g, 0,448 mole) dans 100 ml de toluène en présence de 2 ml d'acide sulfurique concentré. L'eau formée est éliminée par azéotropie. La phase organique est lavée 3 fois à l'eau et est séchée sur sulfate de sodium. On élimine l'excès de menthol par distillation sous vide (140°C sous 0,6 hPa). Le résidu est traité au noir animal dans l'isopropanol au reflux. Après filtration, riçage et évaporation du solvant, on obtient 61,8 g (Rendement 76 %) du malonate de di menthyle sous forme d'une huile jaune qui est utilisée telle quelle dans l'étape suivante.

### Deuxième étape : préparation du 4-nitrobenzalmalonate de 1,3-diméthylbutyle :

Dans un ballon équipé d'un Dean Stark surmonté d'un réfrigérant et sous barbotage d'azote, on place le p-nitro benzaldéhyde (21,8 g, 0,144 mole) et le malonate de di menthyle (61 g, 0,16 mole) dans 100 ml de toluène. On y ajoute le catalyseur préparé par avance, l'acide acétique (0,92 ml) et la pipéridine (1,6 ml) en suspension dans 3 ml de toluène. On porte le mélange sous agitation au reflux pendant 9 heures et élimine par l'intermédiaire du Dean Stark l'eau formée. Trois rajouts des mêmes quantités de catalyseur ont été nécessaires. On verse le mélange réactionnel refroidi dans l'eau et extrait au dichlorométhane. La phase organique est lavée à l'eau, puis séchée et concentrée sous pression réduite. L'huile marron rouge obtenue est chromatographiée sur colonne de silice (éluant : Heptane/EtOAc 95:5). On récupère 37 g (Rendement 50%) des fractions propres de 4-nitrobenzalmalonate de di menthyle sous forme d'une huile jaune et utilisée telle quelle dans l'étape suivante.

### Troisième étape : préparation du 4-aminobenzalmalonate de 1,3-diméthylbutyle

Sous agitation et barbotage d'argon, on disperse le dérivé de l'étape précédente (37 g, 0,072 mole) dans 30 ml d'acide acétique et 45 ml d'eau. Le mélange est chauffé à 50°C. On y ajoute le fer (24,4 g, 0,437 mole) par portions sans dépasser une température de 55°C (temps d'introduction 30 minutes). Ensuite, on ajoute goutte à goutte de l'acide acétique (45 ml) sans dépasser une température de 55 °C (temps d'introduction 1 heure 30 minutes). On chauffe 1 heure de plus à 55°C. On refroidit, ajoute de l'eau et extrait 2 fois au dichlorométhane. La phase organique est lavée à l'eau, avec une solution saturée de bicarbonate de sodium, à l'eau puis est séchée sur sulfate de sodium. Après concentration sous pression réduite, on obtient une gomme orangée que l'on purifie par passage sur colonne de silice (éluant : Heptane/EtOAc 90 :10). On récupère 8,6 g (Rendement 25%) des fractions propres de 4-aminobenzalmalonate de di menthyle sous forme d'un solide jaune et utilisé telle quelle dans l'étape suivante.

### Quatrième étape : préparation du dérivé de l'exemple 7 :

Dans un micro ondes CEM Discover, on laisse le mélange intimement mélangé du produit précédent (2 g, 4,1x10-3 mole), de 2,4-dichloro-6-(4'-amino benzoate d'amyle)-s-triazine (première étape de l'exemple 2) (0,73 g, 2,05x10-3 mole) et de bicarbonate de sodium (0,34 g, 4,1x10-3 mole) pendant 30 minutes à une température de 130-140°C et sous une puissance de 300 Watt. On extrait le solide amorphe formé au dichlorométhane. La phase organique est lavée 3 fois à l'eau et est séchée puis concentrée sous pression réduite. L'huile marron obtenue est soumise à une séparation sur colonne de silice (éluant : Heptane/EtOAc 80 :20). On récupère des fractions propres sous forme d'une poudre jaune pâle du dérivé de l'exemple 7 (0,5 g, Rendement 16%) :
UV (Ethanol) :
   λ = 370 nm, E1% = 391; λmax = 340 nm, E1% = 538; λ = 300 nm, E1% = 313.

**EXEMPLES 8 À 10 DE COMPOSITION**

| **INGREDIENTS** | **EX 8 (hors invention)** | **EX 9 (hors invention)** | **EX10 (invention)** |
|---|---|---|---|
| GLYCERYL STEARATE (and) PEG-100 STEARATE SIMULSOL 165 (Seppic) | 1 | 1 | 1 |
| STEARIC ACID STEARINE TP 1200 (Stéarinerie Dubois) | 1.5 | 1.5 | 1.5 |
| POLY DIMETHYLSILOXANE 200 FLUID 350 CS (Dow Corning) | 0.5 | 0.5 | 0.5 |
| CETYL ALCOHOL NACOL 16-98 (Sasol) | 0.5 | 0.5 | 0.5 |
| CETEARYL ALCOHOL (and) CETEARYL GLUCOSIDE MONTANOV 68 (Seppic) | 2 | 2 | 2 |
| CONSERVATEUR | 1 | 1 | 1 |
| TRIETHANOLAMINE | 0.45 | 0.45 | 0.45 |
| C12-15 ALKYL BENZOATE FINSOLV TN (Witco) | 15 | 15 | 15 |
| TRIAZINE 1,3,5/BENZALMALONATE (EXEMPLE 1) | - | - | 4 |
| BUTYL METHOXYDIBENZOYLMETHANE PARSOL 1789 (DSM) | - | 2 | 2 |
| ETHYLHEXYL METOXYCINNAMATE PARSOL MCX (DSM) | 5 | 5 | 5 |
| GLYCERINE PRICERINE 9091 (Uniquema) | 5 | 5 | 5 |
| XANTHAN GUM KELTROL T (NUTRASWEET) | 0.2 | 0.2 | 0.2 |
| ACIDE ETHYLENE DIAMINE TETRACETIQUE, SEL DISODIQUE, 2 H2O (VERSENE NA2) | 0.1 | 0.1 | 0.1 |
| POTASSIUM CETYL PHOSPHATE AMPHISOL K (Givaudan) | 1 | 1 | 1 |
| ACRYLATES/C10-30 ALKYL ACRYLATE CROSSPOLYMER PEMULEN TR-1 (Noveon) | 0.2 | 0.2 | 0.2 |
| ISOHEXADECANE ISOHEXADECANE (BP) | 1 | 1 | 1 |
| TRIETHANOLAMINE | 0.2 | 0.2 | 0.2 |
| EAU | 70,35 | 63,35 | 59,3 |

### Méthode de mesure de la photostabilté

Pour chaque formule, on a préparé 3 échantillons tests et 3 échantillons témoins. On dépose, à la spatule, 2 mg/cm2 de formule sur des plaques de polyméthacrylate de méthyle.

Les plaques tests sont exposées 38 mn au SUN TEST HERAUS muni d'une lampe Xénon ayant un flux UV-A de 9,51. 10-3 W/cm2 et un flux UV-B de 5,43.10-4 W/cm2. Les plaques témoins sont conservées pendant le même temps et à la même température (38-40°C) à l'obscurité.

A l'issue de ce temps, on procède à l'extraction des filtres en immergeant chaque plaque dans 50 g méthanol et en les soumettant aux ultrasons pendant 15 mn pour assurer une bonne extraction. Les solutions obtenues sont analysées par HPLC et spectrophotométrie UV.

Pour chaque formule testée, le taux d' éthylhexyl-méthoxy-cinnamate résiduel après exposition est donné dans le rapport de sa densité optique (DO) dans l'échantillon exposé à sa densité optique (DO) non exposé. On se place aux maxima d'absorption correspondant au éthylhexyl-méthoxy-cinnamate : λₘₐₓ = 310 nm.

Les résultats obtenus sont résumés dans le tableau 1 suivant :

| **% résiduel en filtre UV-B du type cinnamate après 1 heure UVA** | | |
|---|---|---|
| Filtre UVB cinnamate seul à 5% | Filtre UVB cinnamate à 5% et filtre UV-A dibenzoyméthane à 2% | |
| Composition 8 | Composition 9 sans triazine de formule (I) | Composition 10 avec triazine de formule (I) |
| 60% | 47% | 57% |

La perte sous exposition du filtre UV-B du type ester d'acide cinnamique s'accentue considérablement en présence du filtre UV-A du type dibenzoylméthane.

L'ajout de la triazine de formule (I) permet d'améliorer la stabilité chimique du filtre UV-B et de maintenir l'équilibre de protection UVA-UVB.

## Revendications

1. Composition contenant dans un milieu cosmétiquement acceptable au moins un système filtrant UV, **caractérisée par le fait qu'**elle comprend au moins :
(a) au moins un filtre UV-B du type dérivé ester de l'acide cinnamique
(b) au moins un filtre UV-A du type dérivé du dibenzoylméthane et
(c) au moins un composé s-triazine de formule (I) suivante : 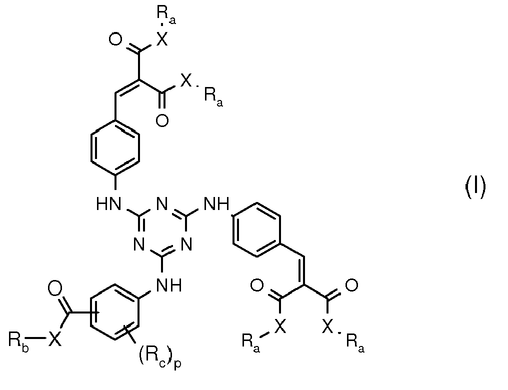
dans laquelle :
X, identiques ou différents, désignent -O- ou -NR₆-
Rₐ, identiques ou différents, désignent un groupe de formule (II) : 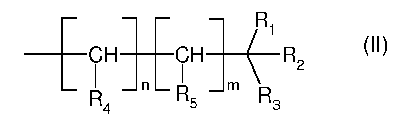
dans laquelle :
R₁ et R₂, identiques ou différents représentent un groupe alkyle en C₁-C₈, linéaire ou ramifié,
R₁ et R₂ peuvent former un cycle en C₅-C₈, éventuellement substitué par 1, 2 ou 3 groupements alkyle(s) en C₁-C₄, linéaire(s) ou ramifié(s) ;
R₃, R₄ et R₅, identiques ou différents représentent un atome d'hydrogène ou un groupe alkyle en C₁-C₄, linéaire ou ramifié ;
n vaut 0 ou 1;
m vaut 0 ou 1 ;
sous réserve que :
(i) lorsque n = 1 et R₄ désigne l'hydrogène alors m est égal à 0 et R₃ est différent d'hydrogène ;
(ii) lorsque R₁ et R₂ forment un cycle en C₅-C₈ alors la somme n+m est différente de 2 ;
R₆ représente l'hydrogène ou un groupe alkyle en C₁-C₈,
R_{b} désigne un groupe alkyle en C₁-C₂₀, linéaire ou ramifié et éventuellement insaturé, un groupe cycloalkyle en C₅-C₁₂, éventuellement substitué par 1 à 3 radicaux alkyles en C₁-C₄, linéaires ou ramifiés, le groupe -(CH₂CHR₇O)_{q}R₈ ou le groupe -CH₂-CH(OH)-CH₂-O-R₈,
R₇ représente l'hydrogène ou méthyle,
R₈ représente l'hydrogène ou un groupe alkyle en C₁-C₈,
q = 1-20,
le groupement COXR_{b} peut être en position ortho, méta ou para du groupement amino,
R_{c} désigne un radical alkyle en C₁-C₂₀, linéaire ou ramifié, saturé ou insaturé, le radical OH, un radical alcoxy en C₁-C₂₀, linéaire ou ramifié,
p est égal à 0, 1 ou 2.

2. Composition selon la revendication 1, où les composés de formule (I) sont choisis parmi ceux pour lesquels les deux conditions suivantes sont réunies :
(a) n=m=0 et
(b) R₁, R₂, R₃ désignent un alkyle en C₁-C₄ et plus particulièrement méthyle ou bien R₃ désigne hydrogène et R₁ et R₂ forment un cycle en C₅-C₈ éventuellement substitué par 1 ou deux radicaux alkyle et plus particulièrement cyclohexyle.

3. Composition selon la revendication 1, où les composés de formule (I) sont choisis parmi ceux pour lesquels les deux conditions suivantes sont réunies :
(a) n=1 et R₄ désigne un alkyle en particulier méthyle ou m = 1 et R₅ désigne un alkyle en particulier méthyle et
(b) R₁ et R₂ désignent un alkyle en C₁-C₄ et plus particulièrement méthyle.

4. Composition selon l'une quelconque des revendications 1 à 3, où les composés de formule (I) sont choisis parmi les composés de formules (1) à (10) suivantes :

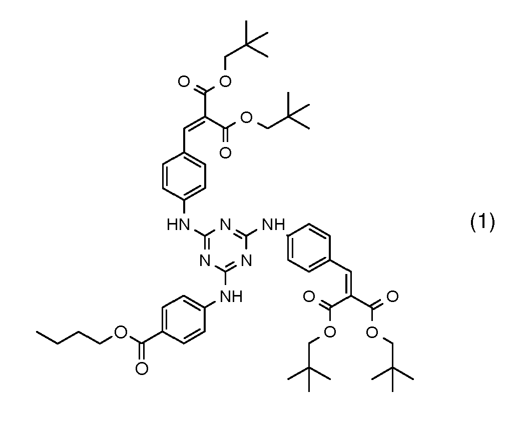

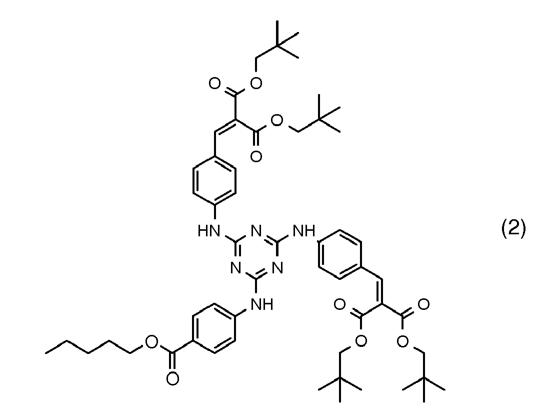

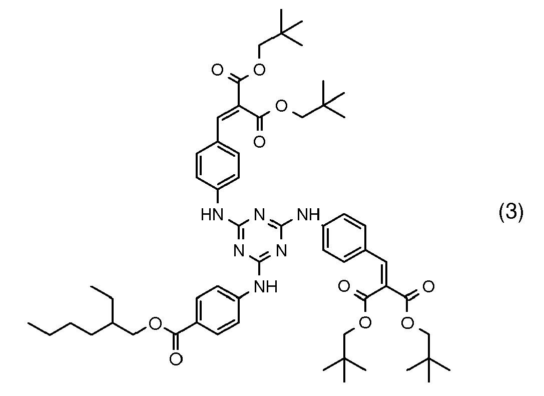

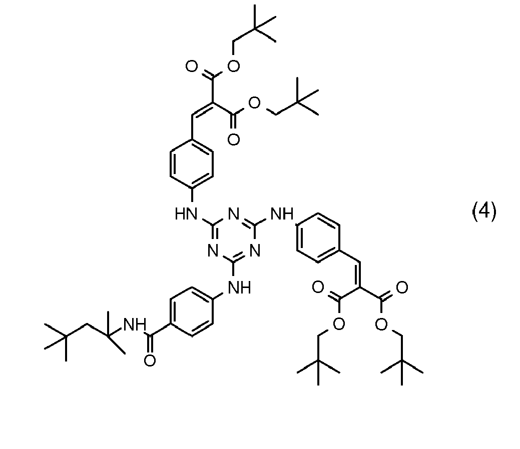

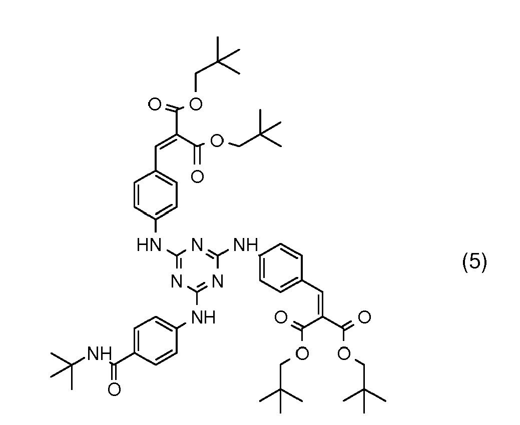

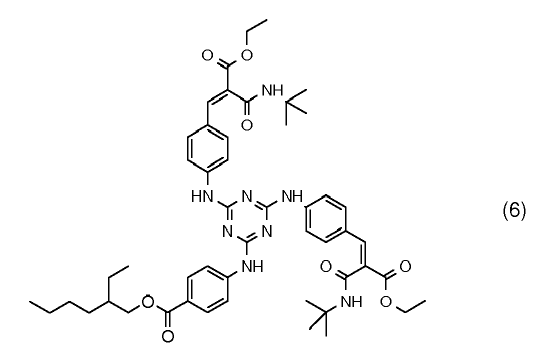

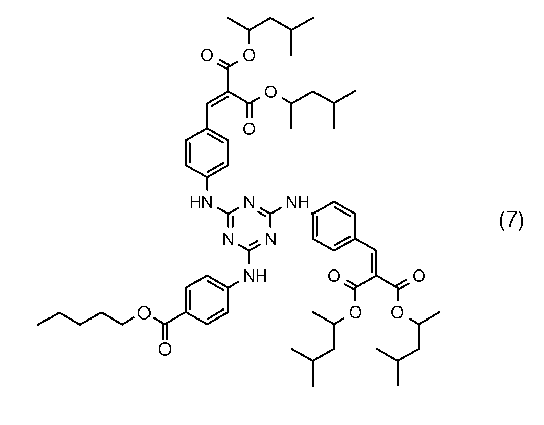

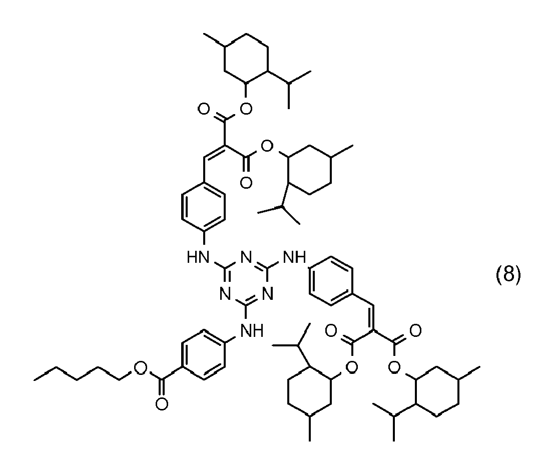

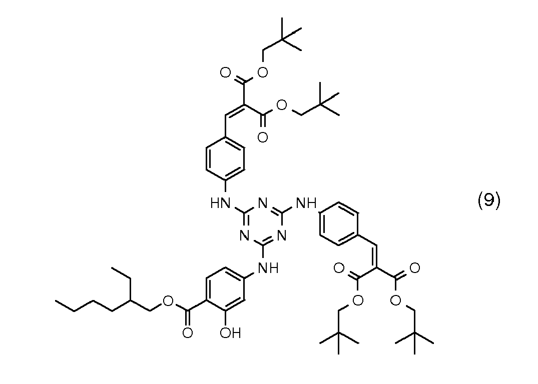

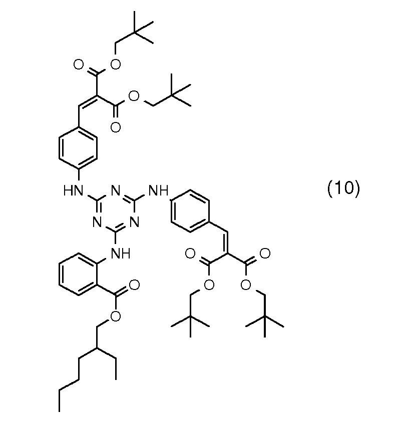

5. Composition selon la revendication 4, dans lequel on utilise comme composé de formule (I) le 2,4-bis-(4'-amino benzalmalonate de di-néopentyle)-6-(4"-amino benzoate de butyle)-s-triazine de formule (1).

6. Composition selon l'une quelconque des revendications précédentes, où le dérivé ester de l'acide cinnamique est choisi parmi ceux répondant à la formule (A) suivante : 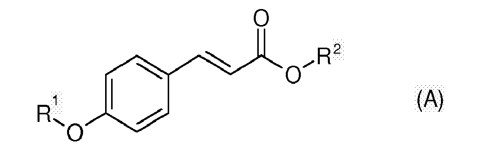 dans laquelle :
R¹, R² sont, indépendamment l'un de l'autre, un radical alkyle en C₁-C₂₄, linéaire ou ramifié et plus particulièrement un radical alkyle en C₁-C₈, linéaire ou ramifié.

7. Composition selon la revendication 6, où le dérivé ester de l'acide cinnamique de formule (A) est choisi parmi :
- Ethylhexyl Methoxycinnamate ou Cinoxate
- Isopropyl Methoxy cinnamate,
- Isoamyl Methoxy cinnamate,
- Diisopropyl Methylcinnamate,

8. Composition selon la revendication 7, où le dérivé ester de l'acide cinnamique de formule (A) est Ethylhexyl Methoxycinnamate ou Cinoxate.

9. Composition selon l'une quelconque des revendications 1 à 8, où le ou les dérivés esters de l'acide cinnamique sont présents à des teneurs qui varient de 0,01 à 20% en poids et plus préférentiellement de 0,1 à 10% en poids et encore plus préférentiellement de 0,1 à 8% en poids par rapport au poids total de la composition.

10. Composition selon l'une quelconque des revendications précédentes, où le dérivé de dibenzoylméthane est choisi parmi :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoyl méthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

11. Composition selon la revendication 10, où le dérivé de dibenzoylméthane est le 4-isopropyl-dibenzoylméthane répondant à la formule suivante :

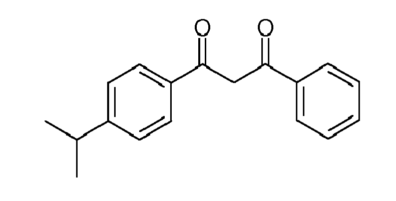

12. Composition selon la revendication 10, où le dérivé de dibenzoylméthane est le 4-(ter.-butyl) 4'-méthoxy dibenzoylméthane ou Butyl Methoxy Dibenzoylmethane de formule suivante :

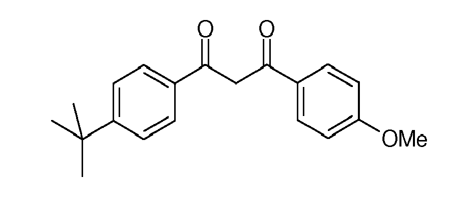

13. Composition selon l'une quelconque des revendications 1 à 12, où le ou les dérivés de dibenzoylméthane sont présents à des teneurs qui varient de 0,01 à 20% en poids et plus préférentiellement de 0,1 à 10% en poids et encore plus préférentiellement de 0,1 à 6% en poids par rapport au poids total de la composition.

14. Composition selon l'une quelconque des revendications 1 à 13, où le ou les composés de formule (I) sont présents dans des proportions allant de 0,01 % à 20 % en poids, de préférence de 0,1 % à 10 % en poids, par rapport au poids total de la composition.

15. Composition selon l'une quelconque des revendications 1 à 14, **caractérisée par le fait qu'**elle contient en plus d'autres agents photoprotecteurs organiques ou inorganiques actifs dans l'UV-A et/ou l'UV-B hydrosolubles ou liposolubles ou bien insolubles dans les solvants cosmétiques couramment utilisés.

16. Composition selon la revendication 15, où les agents photoprotecteurs organiques complémentaires sont choisis parmi les anthranilates ; les dérivés salicyliques, les dérivés du camphre ; les dérivés de la benzophénone ; les dérivés de β,β-diphénylacrylate ; les dérivés de triazine autres que celles de formule (I) ; les dérivés de benzotriazole ; les dérivés de benzalmalonate autres que ceux de formule (I) ; les dérivés de benzimidazole ; les imidazolines ; les dérivés bis-benzoazolyle ; les dérivés de l'acide p-aminobenzoïque (PABA) autres que ceux de formule (I) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

17. Composition selon la revendication 16, **caractérisée par le fait que** le ou les filtres UV organiques sont choisis parmi les composés suivants :
Ethylhexyl Salicylate,
Homosalate,
Octocrylene,
Phenylbenzimidazole Sulfonic Acid,
Disodium Phenyl Dibenzimidazole Tetra-sulfonate,
Benzophenone-3,
Benzophenone-4,
Benzophenone-5,
Diethylamino Hydroxybenzoyl Hexyl Benzoate.
4-Methylbenzylidene camphor,
Terephthalylidene Dicamphor Sulfonic Acid,
Bis-Ethylhexyloxyphenol Methoxyphenyl Triazine
Ethylhexyl Triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
et leurs mélanges.

18. Composition selon la revendication 15, **caractérisée par le fait que** les agents photoprotecteurs inorganiques complémentaires sont des pigments d'oxydes métalliques, traités ou non et plus particulièrement des pigments ayant une taille moyenne des particules primaires entre 5 nm et 100 nm.

19. Composition selon la revendication 18, **caractérisée par le fait que** lesdits pigments sont choisis parmi les oxydes de titane, de zinc, de fer, de zirconium, de cérium et leurs mélanges, traités ou non.

20. Composition selon l'une quelconque des revendications 1 à 19, **caractérisée par le fait qu'**elle comprend en outre au moins un agent de bronzage et/ou de brunissage artificiel de la peau.

21. Composition selon l'une quelconque des revendications 1 à 20, **caractérisée par le fait qu'**elle comprend en outre au moins un adjuvant choisi parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants

22. Composition selon l'une quelconque des revendications 1 à 21, **caractérisée par le fait qu'**elle se présente sous la forme d'une émulsion huile-dans-eau ou eau-dans huile.

23. Utilisation d'une composition telle que définie dans l'une quelconque des revendications 1 à 21 pour la fabrication de produits pour le traitement cosmétique de la peau, des lèvres, des ongles, des cheveux, des cils, sourcils et/ou du cuir chevelu

24. Utilisation d'une composition telle que définie dans l'une quelconque des revendications 1 à 21 pour la fabrication de produits de soin de la peau, des lèvres, des ongles, des cheveux et/ou du cuir chevelu.

25. Utilisation d'une composition telles que définie dans l'une quelconque des revendications 1 à 21 pour la fabrication de produits de maquillage.

26. Procédé pour améliorer la stabilité chimique vis-à-vis du rayonnement UV d'au moins un filtre UV-B du type dérivé ester de l'acide cinnamique en présence d'un filtre UV-A du type dérivé de dibenzoylméthane consistant à associer à ce mélange de filtres UV au moins un composé s-triazine de formule (I) tel que défini dans les revendications précédentes.

27. Utilisation d'un composé s-triazine de formule (I) tel que défini dans les revendications précédentes dans une composition comprenant dans un support cosmétiquement acceptable, l'association d'au moins un filtre UV-B du type dérivé ester de l'acide cinnamique et au moins un filtre UV-A dérivé du dibenzoylméthane telle que définie dans les revendications précédentes, dans le but de d'améliorer la stabilité chimique vis-à-vis du rayonnement UV dudit filtre UV-B.

28. Utilisation d'un composé s-triazine de formule (I) tel que défini dans les revendications précédentes dans une composition comprenant dans un support cosmétiquement acceptable, l'association d'au moins un filtre UV-B du type dérivé ester de l'acide cinnamique et au moins un filtre UV-A dérivé du dibenzoylméthane telle que définie dans les revendications précédentes, dans le but de d'améliorer l'efficacité de ladite composition vis-à-vis des rayons UV-B.

29. Utilisation d'un composé s-triazine de formule (I) tel que défini dans les revendications précédentes dans une composition comprenant dans un support cosmétiquement acceptable, l'association d'au moins un filtre UV-B du type dérivé ester de l'acide cinnamique et au moins un filtre UV-A dérivé du dibenzoylméthane telle que définie dans les revendications précédentes, dans le but de maintenir l'équilibre de protection UVA-UVB durant l'exposition solaire.
